# EUROPEAN PATENT SPECIFICATION

(11) **EP 3 946 481 B1**
(45) Date of publication and mention of the grant of the patent: **04.03.2026**
(21) Application number: 20765328.8
(22) Date of filing: 27.03.2020
(51) Int. Cl.: A61L 26/00, A01N 59/00

(54) **ANTIMICROBIAL COMPOSITIONS**
ANTIMIKROBIELLE ZUSAMMENSETZUNGEN
COMPOSITIONS ANTIMICROBIENNES

(30) Priority: 27.03.2019 GB 201904252; 16.03.2020 GB 202003777
(43) Date of publication of application: 09.02.2022
(73) Proprietor: Matoke Holdings Limited, Abingdon, Oxfordshire OX13 5HR (GB)
(72) Inventor: GREEN, Stewart, Oxfordshire OX13 5HR (GB); BROUGHTON, Mark, Oxfordshire OX13 5HR (GB); RICHINGS-BARROW, Wendy, Oxfordshire OX13 5HR (GB); HARDING, Ian, Oxfordshire OX13 5HR (GB)
(74) Representative: Reddie & Grose LLP
(86) International application number: PCT/GB2020/050837
(87) International publication number: WO 2020/193993

(56) References cited:
- WO-A1-2016/083798
- WO-A1-2018/065608
- WO-A1-2019/077335

## Description

This invention relates to compositions for generating hydrogen peroxide, and their use in treating infections and wounds.

Honey has been used for treatment of microbial infections since ancient times. In recent years there has been a resurgence of interest in the therapeutic efficacy of honey, particularly in the area of wound healing. Clinical trials have shown that honey is an effective broad-spectrum antimicrobial agent which is effective against common wound-infecting organisms, such as *Pseudomonas aeruginosa, Staphylococcus aureus, Candida albicans and Escherichia coli,* and is effective against antibiotic-resistant strains of bacteria. As a natural product, honey also offers an attractive alternative to drug-based treatments.

Many different types of honey have antimicrobial activity. This activity is attributed largely to osmolarity, pH, hydrogen peroxide production and the presence of phytochemical components.

The applicant has appreciated that the antimicrobial effects of honey can be greatly enhanced and controlled by adding glucose oxidase to honey, and that compositions comprising honey and added glucose oxidase are applicable in the treatment of a number of infections, and notably in the treatment of infections caused by biofilms (see WO 2015/166197, WO 2016/083798 and WO 2016/124926).

However, because honey is a natural product, its composition can vary greatly depending on its source. For example, the difference in antimicrobial potency among honeys can be more than one hundred-fold, depending on the geographical, seasonal and botanical source of the honey, as well as the harvesting, processing and storage conditions. Consequently, the antimicrobial efficacy may also vary depending on the type of honey used. Furthermore, honey may also contain other components, such as allergens e.g. trace amounts of pollen, which may cause adverse reactions when applied to certain subjects and make it unsuitable for certain pharmaceutical applications.

Honey may also require processing such that it is in a suitable form for application to subjects, which can add cost and complexity to the production process. Such processing may include creaming or pasteurisation.

WO 2018/065608 discloses compositions for generating antimicrobial activity which comprise an enzyme that is able to convert a substrate to release hydrogen peroxide, a substrate for the enzyme, and a solute in the form of a sugar or sugar derivative having a solubility of at least 100g/100g water at 20°C and 1 atm. The compositions may not comprise sufficient free water to allow the enzyme to convert the substrate.

WO 2018/065789 discloses compositions for generating antimicrobial activity. The compositions comprise an enzyme that is able to convert a substrate to release hydrogen peroxide, a substance that includes a substrate for the enzyme. The compositions may be sterile. The compositions could be used to treat oral or nasal conditions.

WO 2019/077335 discloses a composition that has an enzyme that is able to convert a substrate to release hydrogen peroxide; a substrate for the enzyme; and a superabsorbent component, such as a superabsorbent polymer. The composition is in the form of a powder and may form a gel on contact with water.

Consequently, there is a desire to provide improved compositions which provide enhanced antimicrobial efficacy compared to honey, and which also overcome some of honeys disadvantages. There is also a desire to provide compositions with improved stability and which have the ability to provide antimicrobial activity over an extended period of time.

The scope of the invention is defined by the claims. Any references in the description to methods of treatment refer to the products of the present invention for use in a method for treatment.

There is provided a composition comprising an enzyme that is able to convert a substrate to release hydrogen peroxide and a substrate for the enzyme.

There is also provided a method of making a composition comprising combining an enzyme that is able to convert a substrate to release hydrogen peroxide and a substrate for the enzyme.

According to the invention, there is provided a liquid or gel composition as defined in independent claim 1, and a method of preparing a composition as defined in independent claim 13.

References herein to "enzyme" encompass one or more enzymes. For example, in some embodiments, compositions of the invention may comprise a plurality of enzymes that are able to convert a substrate to release hydrogen peroxide. In some embodiments, compositions of the invention may comprise only one enzyme that is able to convert a substrate to release hydrogen peroxide.

Preferably, the enzyme is a purified enzyme. The term "purified enzyme" is used herein to include an enzyme preparation in which the enzyme has been separated from at least some of the impurities originally present when the enzyme was produced. Preferably impurities that have been removed or reduced include those that would otherwise interfere with the ability of the enzyme to convert the substrate to release hydrogen peroxide.

It may not always be necessary or desirable that the purified enzyme is at a high level of purity provided that the enzyme is able to convert the substrate to release hydrogen peroxide. In some circumstances, it may be desirable to use a relatively crude enzyme preparation. Examples of suitable purity levels include at least 10%, 20%, 30%, 40%, 50%, 60%, 70%, 80%, or 90% pure (mass purity). Preferably, the enzyme is at least 95% pure. Even more preferably, the enzyme is at least 98% pure. Most preferably, the enzyme is at least 99% pure.

The enzyme may have been produced by recombinant or non-recombinant means, and may be a recombinant or non-recombinant enzyme. The enzyme may be purified from a microbial source, preferably from a non-genetically modified microbe.

The level of purity of the enzyme may be selected as appropriate depending on the intended use of the composition. For medical use, a medical grade or medical device grade of purity should be used. For pharmaceutical use, a pharmaceutical grade of purity should be used.

Compositions of the invention may comprise sufficient enzyme and substrate to provide for release of hydrogen peroxide at a specific level or concentration.

Compositions of the invention may provide for release of hydrogen peroxide at a concentration of at least 150 µM, optionally at one hour, 24 hours, or 72 hours following a 1:1 (by weight) dilution of the composition with water.

Compositions of the invention may provide for release of hydrogen peroxide at a concentration of at least 250 µM, optionally at one hour, 24 hours, or 72 hours following a 1:1 (by weight) dilution of the composition with water.

Compositions of the invention may provide for release of hydrogen peroxide at a concentration of at least 500 µM, optionally at one hour, 24 hours, or 72 hours following a 1:1 (by weight) dilution of the composition with water.

Compositions of the invention may provide for release of hydrogen peroxide at a concentration of at least 750 µM, optionally at one hour, 24 hours or 72 hours, following a 1:1 (by weight) dilution of the composition with water.

Compositions of the invention may provide for release of hydrogen peroxide at a concentration of at least 1,000 µM, optionally at one hour, 24 hours, or 72 hours following a 1:1 (by weight) dilution of the composition with water.

Compositions of the invention may provide for release of hydrogen peroxide at a concentration of at least 1,500 µM, optionally at one hour, 24 hours, or 72 hours following a 1:1 (by weight) dilution of the composition with water.

Compositions of the invention may provide for release of hydrogen peroxide at a concentration of at least 2,000 µM, optionally at one hour, 24 hours, or 72 hours following a 1:1 (by weight) dilution of the composition with water.

Compositions of the invention may provide for release of hydrogen peroxide at a concentration of at least 5,000 µM, optionally at one hour, 24 hours, or 72 hours following a 1:1 (by weight) dilution of the composition with water.

Compositions of the invention may provide for release of hydrogen peroxide at a concentration of at least 10,000 µM, optionally at one hour, 24 hours, or 72 hours following a 1:1 (by weight) dilution of the composition with water.

Compositions of the invention may provide for release of hydrogen peroxide at a concentration of 5,000 µM or less, optionally at one hour, 24 hours, or 72 hours following a 1:1 (by weight) dilution of the composition with water.

Compositions of the invention may provide for release of hydrogen peroxide at a concentration of 2,500 µM or less, optionally at one hour, 24 hours, or 72 hours following a 1:1 (by weight) dilution of the composition with water.

Compositions of the invention may provide for release of hydrogen peroxide at a concentration of 2,000 µM or less, optionally at one hour, 24 hours, or 72 hours following a 1:1 (by weight) dilution of the composition with water.

Compositions of the invention may provide for release of hydrogen peroxide at a concentration of 1,000 µM or less, optionally at one hour, 24 hours, or 72 hours following a 1:1 (by weight) dilution of the composition with water.

Compositions of the invention may provide for release of hydrogen peroxide at a concentration of 500 µM or less, optionally at one hour, 24 hours, or 72 hours following a 1:1 (by weight) dilution of the composition with water.

Compositions of the invention may provide for release of hydrogen peroxide at a concentration of 10,000 µM or less, optionally at one hour, 24 hours, or 72 hours following a 1:1 (by weight) dilution of the composition with water.

Compositions of the invention may provide for release of hydrogen peroxide at a concentration of 20,000 µM or less, optionally at one hour, 24 hours, or 72 hours following a 1:1 (by weight) dilution of the composition with water.

Compositions of the invention may provide for release of hydrogen peroxide at a concentration of 30,000 µM or less, optionally at one hour, 24 hours, or 72 hours following a 1:1 (by weight) dilution of the composition with water.

Compositions of the invention may provide for release of hydrogen peroxide at a concentration of 50,000 µM or less, optionally at one hour, 24 hours, or 72 hours following a 1:1 (by weight) dilution of the composition with water.

Compositions of the invention may provide for release of hydrogen peroxide at a concentration of 80,000 µM or less, optionally at one hour, 24 hours, or 72 hours following a 1:1 (by weight) dilution of the composition with water.

Compositions of the invention may provide for release of hydrogen peroxide at a concentration of 100,000 µM or less, optionally at one hour, 24 hours, or 72 hours following a 1:1 (by weight) dilution of the composition with water.

Compositions of the invention may provide for release of hydrogen peroxide at a concentration of 500 to 100,000 µM, optionally at one hour, 24 hours, or 72 hours following a 1:1 (by weight) dilution of the composition with water.

Compositions of the invention may provide for release of hydrogen peroxide at a concentration of 500 to 50,000 µM, optionally at one hour, 24 hours, or 72 hours following a 1:1 (by weight) dilution of the composition with water.

Compositions of the invention may provide for release of hydrogen peroxide at a concentration of 500 to 10,000 µM, optionally at one hour, 24 hours, or 72 hours following a 1:1 (by weight) dilution of the composition with water.

Compositions of the invention may provide for release of hydrogen peroxide at a concentration of 500 to 5,000 µM, optionally at one hour, 24 hours, or 72 hours following a 1:1 (by weight) dilution of the composition with water.

Compositions of the invention may provide for release of hydrogen peroxide at a concentration of 500 to 2,500 µM, optionally at one hour, 24 hours, or 72 hours following a 1:1 (by weight) dilution of the composition with water.

Compositions of the invention may provide for release of hydrogen peroxide at a concentration of 750 to 2,500 µM, optionally at one hour, 24 hours, or 72 hours following a 1:1 (by weight) dilution of the composition with water.

Compositions of the invention may provide for release of hydrogen peroxide at a concentration of 500 to 2,000 µM, optionally at one hour, 24 hours, or 72 hours following a 1:1 (by weight) dilution of the composition with water.

Compositions of the invention may provide for release of hydrogen peroxide at a concentration of 150 to 2,000 µM, optionally at one hour, 24 hours, or 72 hours following a 1:1 (by weight) dilution of the composition with water.

Compositions of the invention may provide for release of hydrogen peroxide at a concentration of 150 to 1,000 µM, optionally at one hour, 24 hours, or 72 hours following a 1:1 (by weight) dilution of the composition with water.

Compositions of the invention may provide for release of hydrogen peroxide at a concentration of 150 to 500 µM, optionally at one hour, 24 hours, or 72 hours following a 1:1 (by weight) dilution of the composition with water.

The level of hydrogen peroxide produced on dilution of compositions of the invention may be sustained at particular levels for a period of time. For example, the level of hydrogen peroxide may be 200 µM to 5,000 µM, preferably at least 400 µM to 2,000 µM for at least 72 hours following dilution of the composition e.g. following a 1:1 (by weight) dilution of the composition with water.

Compositions of the invention may comprise sufficient enzyme and substrate to provide for sustained release of hydrogen peroxide at a level of less than 3 mmol/litre for a period of at least twenty four hours following dilution of the composition, e.g. following a 1:1 (by weight) dilution of the composition with water.

Compositions of the invention may comprise sufficient enzyme and substrate to provide for sustained release of hydrogen peroxide at a level of less than 2 mmol/litre for a period of at least twenty four hours following dilution of the composition, e.g. following a 1:1 (by weight) dilution of the composition with water.

Compositions of the invention may comprise sufficient enzyme and substrate to provide for sustained release of at least 0.2, 0.3, 0.4, 0.5, 1 or 1.5 mmol/litre hydrogen peroxide for a period of at least 24 hours, more preferably 48 hours, optionally following dilution of the composition, e.g. following a 1:1 (by weight) dilution of the composition with water.

So, in some embodiments, compositions of the invention may comprise sufficient enzyme and substrate to provide for sustained release of 0.2 to 3 mmol/litre hydrogen peroxide for a period of at least 24 hours, more preferably 48 hours, e.g. following a 1:1 (by weight) dilution of the composition with water.

In some embodiments, compositions of the invention may comprise sufficient enzyme and substrate to provide for sustained release of 0.3 to 2 mmol/litre hydrogen peroxide for a period of at least 24 hours, more preferably 48 hours, e.g. following a 1:1 (by weight) dilution of the composition with water.

In some embodiments, compositions of the invention may have a particular hydrogen peroxide release profile following dilution. For instance, upon dilution, there may be an initial burst of hydrogen peroxide release, followed by a lower release rate sustained for at least 72 hours. In one example, the composition may provide for release of hydrogen peroxide at a concentration of 1500 µM to 2500 µM at one hour following dilution (e.g. a 1:1, by weight dilution of the composition with water), 1000 µM to 2000 µM at 24 hours following dilution and then 200 µM to 1000 µM between 48 hours and 72 hours following dilution. In another example, the composition may provide for release of hydrogen peroxide at a concentration of about 2000 µM at one hour following dilution (e.g. a 1:1, by weight dilution of the composition with water), 1200 µM to 1500 µM at 24 hours following dilution and then 400 µM to 700 µM between 48 hours and 72 hours following dilution.

The levels of hydrogen peroxide described herein, prior to and/or following dilution may be assessed at ambient temperature, such as normal temperature and pressure (NTP; 20ºC and 1 atm).

Levels of hydrogen peroxide may be established using a method as described in Example 1. Alternatively, levels of hydrogen peroxide may be quantified following the method of Kerkvliet 1996 and Serrano et al., 2004, using Merckoquant test strip (no. 10011; Merck, Germany).

In some embodiments, the composition may not comprise sufficient free water to allow the enzyme to convert the substrate. If the composition does not comprise sufficient free water to allow the enzyme to convert the substrate, hydrogen peroxide production may only occur once it has been diluted by water and there is sufficient free water to allow the enzyme to convert the substrate. Addition of water may thus initiate hydrogen peroxide production.

The skilled person would understand a composition that does not comprise sufficient free water to allow the enzyme to convert the substrate, encompasses a composition that contains a trace amount (or low levels) of free water that may allow a trace amount (or low levels) of hydrogen peroxide to be produced.

Before dilution, hydrogen peroxide may be present at a concentration of 6 ppm or less, 5 ppm or less, 3 ppm or less, or 2 ppm or less. Hydrogen peroxide may be present in the composition at a concentration of 120 µM or less, preferably 100 µM or less, more preferably 80 µM or less. Low levels of hydrogen peroxide before dilution may advantageously improve the shelf life of compositions of the invention. Higher levels of hydrogen peroxide may result in loss of enzyme activity over time. This may be caused by oxidative damage to the enzyme by the hydrogen peroxide being produced.

Once the composition is diluted, hydrogen peroxide may be generated at substantial concentrations. At 1 hour, following a 1:1 dilution (by weight) with water, the level of hydrogen peroxide production may increase by a factor of at least 5, at least 10, at least 20, at least 50, at least 100, or at least 200. At 24 hours, following a 1:1 dilution (by weight) with water, the level of hydrogen peroxide production may increase by a factor of at least 5, at least 10, at least 20, at least 50, at least 100, or at least 200.

In compositions of the invention, the water activity (a_{w}) may be 0.7 or less, or 0.6 or less. For example, the water activity may be 0.3 to 0.7 or 0.4 to 0.6. A low water activity may be advantageous in preventing microbial proliferation, and it may be advantageous in minimising hydrogen peroxide production prior to activation by dilution.

Water activity is typically measured using a hygrometer, such as a resistive electrolytic hygrometer, a capacitance hygrometer or a dew point hygrometer. Measurement of water activity would typically take place at ambient temperature, such as normal temperature and pressure. Measurement of water activity may take place according to ISO 18787:2017. Suitable amounts of water will vary depending on the precise components of the composition. Typically, there would be 25% (by weight) or less, preferably 20% or less (by weight) water in the composition, for example, 5%-20%, water. In one embodiment, there is 5 to 17% (by weight) of water. In one embodiment, there is 5 to 15% (by weight) of water. In a preferred embodiment, the amount of water in the composition is 10% or less, preferably 7.5% or less, more preferably 5% or less (by weight). In one embodiment, the amount of water may be 1% to 10% (by weight), such as 2.5% to 7.5% (by weight). In some embodiments, the composition may comprise substantially no water, or only trace amounts of water.

Compositions of the invention are preferably fluid at normal temperature and pressure (20°C and 1 atm). For example, a composition of the invention may be a solution. Preferably, the composition comprises substantially no crystalline or solid phase. Preferably, the composition is not a supersaturated solution. The solvent may comprise water and/or a non-aqueous solvent. Inclusion of a non-aqueous solvent may provide certain additional benefits, such as improving the stability of the composition.

Compositions of the invention may comprise 25 to 2000 ppm of the enzyme, for example 50 to 1000 ppm of the enzyme. Compositions of the invention may comprise 750 to 2000 ppm of the enzyme. Compositions of the invention may comprise greater than 500 ppm of the enzyme. Compositions of the invention may comprise 250 to 1500 ppm of the enzyme.

The enzyme may be 0.0005% to 0.5% (by weight), 0.001% to 0.2% (by weight), 0.001% to 0.1% (by weight) enzyme, or 0.01% to 0.05% (by weight) of the composition. The enzyme may be 0.001% to 0.01% (by weight) of the composition.

The enzyme activity (for example, the glucose oxidase activity) may range, for example, from 1-400 IU/mg, or 1-300 IU/mg, for example 250-280 IU/mg. The amount of enzyme used is likely to depend on several factors, including the desired use of the composition, the desired level of hydrogen peroxide release, and the desired length of time for hydrogen peroxide release. A suitable amount of enzyme can readily be determined by a person of ordinary skill in the art, if necessary using a well diffusion assay, to determine the extent of hydrogen peroxide release for different amounts of enzyme. The amount of enzyme used may be selected so as to produce a composition for generating antimicrobial activity that is equivalent to a selected phenol standard (for example a 10%, 20%, or 30% phenol standard).

Compositions of the invention may comprise at least 1 unit, and preferably up to 1500 units, of the enzyme per gram of the composition. A "unit" is defined herein as the amount of enzyme (e.g. glucose oxidase) causing the oxidation of 1 micromole of substrate (e.g. glucose) per minute at 25 degrees centigrade at pH 7.0.

Preferably, the enzyme is, or comprises, an oxidoreductase enzyme. Examples of oxidoreductase enzymes include glucose oxidase, hexose oxidase, cholesterol oxidase, galactose oxidase, pyranose oxidase, choline oxidase, pyruvate oxidase, glycollate oxidase, amino acid oxidase, or mannose oxidase.

Preferably, the oxidoreductase enzyme is glucose oxidase and the substrate for the oxidoreductase enzyme is glucose.

In some embodiments, a composition according to the invention comprises more than 15 units, for example at least 30 units, at least 50 units, or at least 100 units, and suitably less than 685 units, for example 100-500 units, of enzyme (e.g. glucose oxidase) per gram of the composition. In other embodiments of the invention, a composition according to the invention comprises at least 500 units, for example 500-1000 units, or 685-1000 units, of enzyme (e.g. glucose oxidase) per gram of the composition.

References herein to "substrate" encompass one or more substrates. For example, in some embodiments, compositions of the invention may comprise a plurality of substrates. In some embodiments, compositions of the invention may comprise only one substrate.

Preferably, the substrate is a purified substrate. The term "purified substrate" is used herein to include a substrate which has been separated from at least some of the impurities originally present when the substrate was obtained or produced. The purified substrate may be obtained from a natural source or may be synthetically produced. The purified substrate may be a processed, extracted, or refined substrate (i.e. a substrate in which impurities or unwanted elements have been removed by processing).

It may not always be necessary or desirable that the purified substrate is at a high level of purity provided that the enzyme is able to convert the substrate to release hydrogen peroxide. In some circumstances, it may be desirable to use a relatively crude substrate. Examples of suitable purity levels include at least 10%, 20%, 30%, 40%, 50%, 60%, 70%, 80%, 90%, 95%, or 99% pure (mass purity). Preferably the purity level is at least 90%. Even more preferably, the purity is at least 99%. However, in some embodiments, it may be desirable that the purified substrate is a medical grade, medical device grade, or pharmaceutical grade substrate.

In particular embodiments, the substrate is, or comprises, sugar. The term "sugar" is used herein to refer to a carbohydrate with the general formula Cₘ(H₂O)ₙ. The purified sugar may be obtained from a natural source (for example a processed, extracted, or refined natural sugar), or be synthetically produced. The sugar may be at least 10%, 20%, 30%, 40%, 50%, 60%, 70%, 80%, 90%, 95%, or 99% pure (mass purity). Preferably the purity level is at least 90%. Even more preferably, the purity is at least 99%. The sugar may be a medical grade, medical device grade, or pharmaceutical grade sugar. The sugar may be a monosaccharide or a disaccharide, preferably a monosaccharide. The sugar may include, for example purified D-glucose, hexose, or D-galactose. For example, the purified sugar may be medical grade, medical device grade, or pharmaceutical grade D-glucose, hexose, or D-galactose.

Instead of, or in addition to, the substrate, the composition may comprise a precursor substrate. Any disclosure herein which relates to the substrate, such as amounts and purity, may also apply to the precursor substrate.

For compositions of the invention which comprise a precursor-substrate, the composition may comprise one or more enzymes for converting the precursor-substrate to the substrate for the enzyme. However, in some embodiments, the precursor-substrate may not necessarily be converted to the substrate enzymatically. For example, for some precursor substrates, addition of water may be sufficient for conversion. Alternatively or additionally, compositions of the invention may comprise non-enzymatic catalysts.

Compositions which comprise a precursor-substrate may comprise a first enzyme that is able to convert the substrate to release hydrogen peroxide, and a second enzyme that is able to convert the precursor-substrate to the substrate for the first enzyme.

The precursor-substrate is preferably a carbohydrate, such as a polysaccharide, or a sugar e.g. a disaccharide, or sugar derivative. For example, the precursor-substrate may be sucrose, the first enzyme may be glucose oxidase and the second enzyme may be invertase. **In** another example, the precursor-substrate may be maltose, the first enzyme may be glucose oxidase and the second enzyme may be maltase.

Compositions of the invention which comprise a precursor-substrate may comprise an enzyme (preferably a purified enzyme) that is able to convert the substrate to release hydrogen peroxide, and at least two enzymes (e.g. second and third enzymes, preferably purified enzymes) that are able to convert the precursor-substrate to the substrate for the first enzyme. For example, the precursor-substrate may be starch, the first enzyme may be glucose oxidase and the second and third enzymes may be amylase and maltase. For example, the precursor-substrate may be cellulose, the first enzyme may be glucose oxidase and the second and third enzymes may be cellulose and beta-glucosidase.

Compositions of the invention preferably comprise an additional component in the form of solute. References herein to "solute" encompass one or more solutes. For example, in some embodiments, compositions of the invention may comprise a plurality of solutes. In some embodiments, the composition may comprise only one solute. Preferably, the solute is soluble in water.

The solute may be distinct from the substrate, or in some examples, the substrate may be the same as the solute. For example, the composition may comprise fructose and fructose oxidase: the fructose being both the solute and the substrate for enzyme. In preferred embodiments, the solute is different from the substrate. In one particularly preferred embodiment, the substrate is glucose and the solute is fructose.

The solute is preferably purified, meaning that the solute has been separated from at least some of the impurities originally present when the solute was obtained or produced. The solute may be obtained from a natural source or may be synthetically produced. The solute may be a processed, extracted, or refined substrate (i.e. a solute in which impurities or unwanted elements have been removed by processing). It may not always be necessary or desirable that the solute is at a high level of purity. In some circumstances, it may be desirable to use a relatively crude solute preparation. Examples of suitable purity levels include at least 10%, 20%, 30%, 40%, 50%, 60%, 70%, 80%, 90%, 95%, or 99% pure (mass purity). Preferably, the purity level is at least 90%. More preferably, the purity is at least 99%. However, in some embodiments, it may be desirable that the solute is a medical grade, medical device grade, or pharmaceutical grade solute.

In a preferred embodiment, the enzyme is at least 95% pure, the solute is at least 95% pure and the substrate is at least 95% pure (all with reference to mass purity).

In another preferred embodiment, the enzyme is at least 98% pure, the solute is at least 98% pure and the substrate is at least 98% pure (all with reference to mass purity).

In another preferred embodiment, the enzyme is at least 99% pure, the solute is at least 99% pure and the substrate is at least 99% pure (all with reference to mass purity).

Compositions of the invention, or components of compositions of the invention, may be pharmaceutical grade. The term "pharmaceutical grade" is used herein to refer to include reference to a purity standard for a reagent that has been established by a recognized national or regional pharmacopeia (e.g., the U.S. Pharmacopeia (USP), British Pharmacopeia (BP), National Formulary (NF), European Pharmacopoeia (EP), or Japanese Pharmacopeia (JP)).

The solute may be, or may comprise, a carbohydrate. The solute may be, or may comprise, a polysaccharide. Preferably, the solute is, or comprises, sugar or sugar derivative. More preferably, the solute is, or comprises, a sugar. Suitable sugars include oligosaccharides, disaccharides or monosaccharides. Preferably, the sugar is a disaccharide or a monosaccharide. In particularly preferred embodiments, the sugar is a monosaccharide. Suitable sugars may include fructose, glucose, galactose, sucrose, maltose. In a particularly preferred embodiment, the sugar is fructose.

"Sugar derivative" is used herein to refer to a sugar that has been modified by addition of one or more substituents. The substituent may be something other than a hydroxyl group. Sugar derivatives encompasses amino sugars, acidic sugars, deoxy sugars, sugar alcohols, glycosylamines and sugar phosphates. For example, sugar derivatives may include glucose-6-phosphateglucosamine, glucoronate, gluconate, galactosamine, glucosamine, sialic acid, deoxyribosefucose, rhamnose glucuronic acid, polyols (e.g. sorbitol, erythritol, xylitol, mannitol, lactitol and maltitol) and sucralose.

Compositions of the invention may comprise two or more solutes, as described herein. For example, compositions of the invention may comprise two or more sugars or sugar derivatives. The composition may comprise a maximum of two solutes, e.g. two sugars or sugar derivatives; or a maximum of three solutes, e.g. three sugars or sugar derivatives. For instance, a composition of the invention may comprise glucose, fructose and sucrose. However, in a particularly preferred embodiment of the invention, the composition comprises glucose and fructose and no other mono or disaccharides.

If the solute is distinct from the substrate, the amount of the solute in the composition is preferably greater than the amount of the substrate in the composition.

The solute preferably has a high solubility in water, for example a solubility which is greater than glucose. Glucose has a solubility of 90g/100g water at 20°C and 1 atm. In a preferred embodiment, the solute has a solubility greater than or equal to 100g/100g water at 20°C and 1 atm, in a more preferred embodiment, the solute has a solubility greater than or equal to 200g/100g water at 20°C and 1 atm, in an even more preferred embodiment, the solute has a solubility greater than 300g/100g water at 20°C and 1 atm.

A solute with a high solubility may be advantageous because if the composition of the invention is a solution, it may enable the solution to have a relatively high concentration of solute, which may in turn provide a high osmolarity or osmotic strength. Compositions with a high osmolarity or osmotic strength may assist with the antimicrobial efficacy of the composition because they may reduce the amount of water available for microbes or draw water away from microbes, and may assist in wound healing and wound debridement. A solute with a high solubility may also assist in obtaining a desired viscosity.

Fructose is a particularly preferred solute because it has a solubility of about 375g/100g water at 20°C and 1 atm. Consequently, the solute may be fructose.

Surprisingly, the applicant has found that properties of compositions of the invention can be improved by reducing the concentration of substrate. This is counterintuitive, as many existing hydrogen peroxide-generating compositions have focussed on trying to replicate the levels of the sugars present in honey, which typically have levels of glucose well in excess of 20% by weight. For example, a typical blossom honey has a concentration of glucose above 30% by weight. Synthetic compositions with higher levels of glucose similar to those found in honey are described, for example, in WO 2018/065608 (31% glucose), WO 2008/041218 (38%±5 glucose).

The applicant has appreciated that using higher levels of substrate (e.g. glucose) may result in crystallisation. This is particularly disadvantageous for compositions intended to be applied to wounds because it can result in a product with a gritty feel, and one which lacks homogeneity. Furthermore, during storage, crystallisation can take place which can significantly affect the product's shelf-life. Higher levels of substrate may also make the composition difficult to mix effectively during manufacture.

The applicant has also appreciated that inclusion of high levels of substrates such as glucose in compositions for wound treatment could cause detrimental effects. For instance, Xuan YH, et al. (2014) High-Glucose Inhibits Human Fibroblast Cell Migration in Wound Healing via Repression of bFGF-Regulating JNK Phosphorylation. PLoS ONE 9(9): e108182) indicates that studies in diabetic patients implicate high circulating glucose levels with poor wound healing. In particular, the study demonstrated the poor mobilisations of fibroblasts in a cell culture system in the presence of 30mM glucose.

According to compositions of the invention, there is less than 10% by weight of the substrate. There may be 7.5% or less, by weight of the substrate, e.g. about 5%, by weight of the substrate.

There may be at least 1% by weight, at least 2% by weight, at least 2.5% by weight of the substrate, at least 3% by weight of the substrate, or at least 5% by weight of the substrate.

The composition may have 2% to less than 10% by weight of the substrate. The composition may have 2.5% to 7.5% by weight of the substrate.

The solute when different from the substrate may be present in an amount of at least 20% by weight, at least 25% by weight, at least 40% by weight, at least 50% by weight, at least 60% by weight, at least 65% by weight, at least 70% by weight, or at least 75% by weight.

Some compositions of the invention may have lower levels of solute. Therefore, in preferred embodiments, the amount of solute in the composition is 60% or less, 50% or less, 45% or less, 40% or less, 35% or less, 30% or less. In one embodiment, there is 20 to 60% by weight of the solute in the composition. In a more preferred embodiment, there is 25 to 55% by weight of the solute in the composition. In another preferred embodiment, there is 35 to 50% by weight of the solute in the composition.

Honey has a high concentration of sugars. For instance, blossom honey typically has a total sugar content of nearly 80% by weight. The applicant has appreciated that lower total levels of sugar or sugar derivative may further reduce the risk of crystallisation and the unfavourable properties which result from crystallisation. This, again, is contrary to synthetic compositions of the art which are based on recreating the levels of solutes, such as sugars, which naturally occur in honey. WO 2018/065608, for example, discloses compositions with a total sugar content of 83% by weight, with fructose being present in an amount of 52% by weight. WO 2008/041218, for example, discloses synthetic compositions with a total sugar content of 84.5%, including glucose, fructose, maltose and sucrose.

For example, the total content of sugar or sugar derivative in the composition may be 80% or less, 70% or less, 60% or less, 50% or less, 45% or less or 40 % or less (all by weight). To optimise rheological properties and water activity, sugars or sugar derivatives still may be present in a substantial amount, such as at least 20%, at least 25%, at least 30% or at least 35% (by weight). For example, the total content of sugar or sugar derivative in the composition may be 20% to 70% by weight, 25 to 60% by weight, such as 30% to 55% by weight, or 40 to 50% by weight.

The solute may contribute to a desirable viscosity of the composition, it may act as a bulking agent and it may act as a water activity moderator, to ensure that the composition maintains a low water activity and very low levels of hydrogen peroxide prior to dilution. In this respect, fructose may be particularly beneficial.

The total amount of substrate and solute in the composition may be less than 70%, 60% or less, 50% or less, 45% or less or 40 % or less (all by weight). The total amount of substrate and solute may be at least 20%, at least 25%, at least 30% or at least 35% (by weight). For example, the total amount of substrate and solute in the composition may be 20% to less than 70% by weight, 25 to 60% by weight, such as 30% to 50% by weight.

A composition may comprise: enzyme that is able to convert a substrate to release hydrogen peroxide; and substrate for the enzyme; wherein the composition has a water activity of 0.7 or less, wherein there is less than 20%, by weight, of the substrate in the composition and wherein i) the total amount of sugar or sugar derivative in the composition is 25 to 60% by weight and/or ii) the composition comprises solute in the form of a sugar or sugar derivative having a solubility of at least 100g/100g water at 20°C and 1 atm and the total amount of substrate and solute in the composition is 25 to 60% by weight.

A method of preparing a composition may comprise, combining: enzyme that is able to convert a substrate to release hydrogen peroxide; and substrate for the enzyme, wherein the composition is formulated such that it has a water activity of 0.7 or less, such that there is less than 20%, by weight, of the substrate and such that i) the total sugar or sugar derivative content is 25 to 60% by weight and/or ii) the composition comprises solute in the form of a sugar or sugar derivative having a solubility of at least 100g/100g water at 20°C and 1 atm and the total amount of substrate and solute in the composition is 25 to 60% by weight.

Compositions of the invention may comprise at least two sugars or sugar derivatives (e.g. including glucose and fructose). The composition may comprise a maximum of two sugars or sugar derivatives (e.g. only glucose and fructose).

Compositions of the invention may have 1 weight part substrate to 2 weight parts or greater solute. For instance, compositions of the invention may have 1 weight part solute to greater 3 weight parts, or greater, solute; 1 weight part substrate to 5 weight parts, or greater, solute; 1 weight part substrate to greater 10 weight parts, or greater, solute; 1 weight part substrate to 12 weight parts, or greater solute; or 1 weight part substrate to 15 weight parts or greater, solute.

For example, compositions of the invention may have a weight ratio of substrate to solute of 1:50 to 1:4. For example, compositions of the invention may have a weight ratio of substrate to solute of 1:32 to 1:8. For example, compositions of the invention may have a weight ratio of substrate to solute of 1:20 to 1:15.

A composition may comprise: enzyme that is able to convert a substrate to release hydrogen peroxide; substrate for the enzyme; and solute in the form of a sugar or sugar derivative having a solubility of at least 100g/100g water at 20°C and 1 atm, wherein the composition has a water activity of 0.7 or less, wherein the total amount of sugar or sugar derivative in the composition is 25% to 60% by weight, and wherein there is 1 weight part of the substrate to at least 2 weight parts of the solute.

A method of preparing a composition may comprise, combining: enzyme that is able to convert a substrate to release hydrogen peroxide; substrate for the enzyme, and solute in the form of a sugar or sugar derivative having a solubility of at least 100g/100g water at 20°C and 1 atm, wherein the composition is formulated such that it has water activity of 0.7 or less, such that the total amount of sugar or sugar derivative in the composition is 25% to 60% by weight, and such that there is 1 part by weight of the substrate to at least 2 parts by weight of the solute.

Compositions of the invention may be formulated such that there is 1 part by weight of enzyme to 400 parts by weight, or less, of substrate. Preferably, there is 1 part by weight enzyme to 200 parts, or less, by weight of substrate. More preferably, there is 1 part by weight enzyme to 100 parts, or less, by weight of substrate. Even more preferably there is 1 part by weight enzyme to 50 parts, or less, by weight of substrate.

Compositions of the invention may comprise a buffer. An example of a suitable buffer is a citric acid/NaOH buffer. The buffer may be present in a concentration of 2 to 200 µM, such as 5 to 100 µM. The concentration of buffer may be 10 µM, for example.

Compositions of the invention may have a pH of 5 or less, e.g. 3 to 5 (such as about pH 4). Alternatively, compositions of the invention may have a pH greater than 5, e.g. 6 to 8 (such as about pH 7). Compositions of the invention may have a pH of 3.5 to 6, preferably 4.5 to 5.5. In some embodiments, compositions of the invention may have a pH of 5.0 to 7.5.

Compositions of the invention may have a viscosity (dynamic viscosity), of at least 10000 mPas at 20°C and 1 atm. Compositions of the invention may have a viscosity of 10000 to 20000 mPas at 20°C and 1 atm, more preferably 12000 to 15000 mPas at 20°C and 1 atm. A viscous solution or liquid may at least in part be afforded by high concentrations of sugars or sugar derivatives. A high viscosity may be beneficial in allowing the composition to remain in contact with a wound throughout the treatment period. However, the applicant has appreciated that desirable viscosities may be achieved with lower levels of sugars or sugar derivatives. This can be achieved, for example, by including thickeners, viscosity enhancers or gelling agents such as hydrocolloid gelling agents. Typically, viscosity enhancers are polymers, particularly pharmaceutically acceptable polymers such as hydroxymethyl cellulose, carboxymethyl cellulose and carbopol (cross-linked polyacrylic acid polymer). Other suitable thickeners or viscosity enhancers (such as polymers) are described herein. Preferably, the viscosity enhancers are inert.

It is advantageous for compositions of the invention to have rheological properties which permit it to be applied to a wound and remain in effective contact with a wound for the duration of treatment. Although compositions which contain high levels of sugars (e.g. levels similar to those found in honey) can create viscous compositions which can be applied effectively to a wound, the applicant has found that such compositions may be prone to becoming less viscous as they warm up to body temperature, which can decrease it efficacy. By using thickeners or viscosity enhancers and lower levels of sugars or sugar derivatives, the applicant has found that such compositions are or effective at remaining in position on a wound. Furthermore, the lower levels of sugars can reduce the risk of crystallisation, as described above.

A composition may comprise enzyme that is able to convert a substrate to release hydrogen peroxide; substrate for the enzyme; and
viscosity enhancer (preferably polymer), wherein the water activity of the composition is 0.7 or less, and wherein the viscosity of the composition is 12000 to 15000 mPas at 20ºC and 1 atm.

A method of preparing a composition may comprise combining: enzyme that is able to convert a substrate to release hydrogen peroxide; substrate for the enzyme; and viscosity enhancer (preferably polymer), wherein the composition is prepared such that it has a water activity of 0.7 or less, and such that it has viscosity of 12000 to 15000 mPas at 20ºC and 1 atm.

A composition may comprise enzyme that is able to convert a substrate to release hydrogen peroxide; substrate for the enzyme; and
viscosity enhancer (preferably polymer), wherein the water activity of the composition is 0.7 or less, and wherein: i) the total amount of sugar or sugar derivative in the composition is 25 to 60% by weight; and/or ii) the composition comprises solute in the form of a sugar or sugar derivative having a solubility of at least 100g/100g water at 20°C and 1 atm and the total amount of substrate and solute in the composition is 25 to 60% by weight.

A method of preparing a composition may comprise combining: enzyme that is able to convert a substrate to release hydrogen peroxide; substrate for the enzyme; and viscosity enhancer (preferably polymer), wherein the composition is prepared such that it has a water activity of 0.7 or less, and such that: i) the total amount of sugar or sugar derivative in the composition is 25 to 60% by weight; and/or ii) the composition comprises solute in the form of a sugar or sugar derivative having a solubility of at least 100g/100g water at 20°C and 1 atm and the total amount of substrate and solute in the composition is 25 to 60% by weight.

A thickener, viscosity enhancer or gelling agent, such as a polymer, may be present in an amount less than 5%. For example, it may be present in an amount of 0.1% by weight to 2% by weight, preferably 0.2% to 1% by weight. The total amount of polymer in the composition may be 0.1 to 20% by weight, such as 0.2 to 15% by weight. Compositions may comprise a mixture of polymers, or may comprise only one polymer. For example, the composition may comprise carboxymethyl cellulose and polyethylene glycol.

Hydrocolloids are a heterogeneous group of hydrophilic, long-chain polymers (polysaccharides or proteins) characterised by their ability to form viscous dispersions and/or gels when dispersed in water (Saha and Bhattacharya, J Food Sci Technol, 2010, 47(6):587-597). The extent of thickening varies with the type and nature of the hydrocolloid. Some provide low viscosities at a fairly high concentration, but most provide a high viscosity at a concentration below 1%. The viscosity of hydrocolloid dispersions arises predominantly from non-specific entanglement of conformationally disordered polymer chains. Hydrocolloids that can be used as thickening agents (referred to herein as hydrocolloid thickeners) include starch, modified starch, xanthan, galactomannans (such as guar gum, locust bean gum, and tara gum), gum Arabic or acacia gum, gum karaya, gum tragacanth, konjac maanan, and cellulose derivatives such as carboxymethyl cellulose, methyl cellulose, and hydroxypropylmethyl cellulose.

Some hydrocolloids are able to form gels, consisting of polymer molecules cross-linked to form an interconnected molecular network immersed in a liquid medium. A rheological definition of a gel is a viscoelastic system with a 'storage modulus' (G') larger than the 'loss modulus' (G") (de Vries 2004, Gums and stabilizers for the food industry, vol 12. RSC Publ, Oxford, pp 22-30). Hydrocolloids form gels by physical association of their polymer chains through hydrogen bonding, hydrophobic association, and cation-mediated cross-linking. Gelling-type hydrocolloids (or hydrocolloid gelling agents) include alginate, pectin, carrageenan, gelatin, gellan, agar, modified starch, methyl cellulose and hydroxypropylmethyl cellulose.

Gelation of hydrocolloids can occur by different mechanisms: ionotropic gelation, cold-set gelation and heat-set gelation (Burey et al. 2008, Crit Rev Food Sci Nutr 48:361-377). lonotropic gelation occurs via cross-linking of hydrocolloid chains with ions, typically a cation-mediated gelation process of negatively-charged polysaccharides. Examples of hydrocolloids that can form gels by ionotropic gelation include alginate, carrageenan and pectin. Ionotropic gelation can be carried out by either diffusion setting or internal gelation. In cold-set gelation, hydrocolloid powders are dissolved in warm/boiling water to form a dispersion which forms a gel on cooling. Agar and gelatin form gels by this mechanism. Heat-set gels require the application of heat to gel (for example, curdlan, konjac glucomannan, methyl cellulose, starch and globular proteins).

**In** particular embodiments, the hydrocolloid is, or comprises, a polysaccharide or a protein. The hydrocolloid may be a hydrocolloid thickener, such as starch, modified starch, xanthan, a galactomannan (such as guar gum, locust bean gum, and tara gum), gum Arabic or acacia gum, gum karaya, gum tragacanth, konjac maanan, or a cellulose derivative, such as carboxymethyl cellulose, methyl cellulose, or hydroxypropylmethyl cellulose.

In other embodiments, the hydrocolloid is, or comprises a cross-linked hydrocolloid, for example a cross-linked polysaccharide, such as cross-linked alginate, pectin, carrageenan, gelatin, gellan, agar, agarose, modified starch, or a cellulose derivative, such as methyl cellulose or hydroxypropylmethyl cellulose.

The hydrocolloid may be cross-linked by any suitable method, for example including the methods for gelation of hydrocolloids described above: ionotropic gelation, cold-set gelation and heat-set gelation. In particular embodiments, molecules of the hydrocolloid are cross-linked by cations (for example calcium ions) as a result of ionotropic gelation of a hydrocolloid gelling agent. Examples of hydrocolloid cross-linked by cations that may be present in a composition of the invention include alginate, carrageenan or pectin.

In particular embodiments, a composition of the invention includes cross-linked alginate, for example alginate cross-linked by calcium ions. Alginate can form gels without prior heating because sodium alginate is soluble in cold water.

Cross-linked alginate may be formed from sodium alginate and calcium ions (for example, provided by calcium chloride). In some embodiments, water may be used as solvent to dissociate the calcium ions. However, since this could potentially activate production of hydrogen peroxide by the enzyme and the substance that includes a substrate for the enzyme, and limit the stability of the composition, it may be preferred to use a non-aqueous solvent to dissociate the calcium ions, such as ethanol or acetic acid.

Compositions of the invention preferably contain substantially no catalase. Compositions of the invention preferably contain essentially no catalase. In some embodiments, compositions of the invention may comprise catalase.

Compositions of the invention preferably contain substantially no peroxidase. Compositions of the invention preferably contain essentially no peroxidase.

Compositions of the invention preferably contain substantially no lactoferrin. Compositions of the invention preferably contain essentially no lactoferrin.

Compositions of the invention preferably do not comprise an unrefined substance. The term "unrefined" is used herein to refer to substances that have not been processed into a pure form. Unrefined substances include substances that may have been concentrated, for example by drying or boiling.

Compositions of the invention preferably do not include one or more substrates from a natural source (termed herein a "natural substance"). Examples of natural substances include substances from a plant source, including from sap, roots, nectar, flowers, seeds, fruit, leaves, or shoots.

Preferably, compositions of the invention do not comprise an unrefined natural substance. Compositions of the invention preferably do not comprise honey.

Compositions of the invention may comprise additional components that may aid in the stability of the composition, such as the stability of the enzyme. A composition of the invention may thus comprise a protein-stabilising excipient.

A composition of the invention may comprise non-aqueous solvent, such as an organic solvent. In compositions of the invention that comprise non-aqueous solvent, the non-aqueous solvent may comprise ethanol, dimethyl sulphoxide, glycerol, ethylene glycol or propylene glycol. Preferably, the non-aqueous solvent is or comprises a polyol. An example of a particularly suitable polyol is glycerol. The applicant has found that non-aqueous solvents (e.g. polyols such as glycerol) can be effective at providing a stable and effective composition.

Non-aqueous solvent may comprise only one non-aqueous solvent, or it may comprise a plurality of non-aqueous solvents.

A composition may comprise: enzyme that is able to convert a substrate to release hydrogen peroxide; purified substrate for the enzyme; and non-aqueous solvent, the water activity of the composition is 0.7 or less, and wherein: i) the total amount of sugar or sugar derivative in the composition is 25 to 60% by weight; and/or ii) the composition comprises solute in the form of a sugar or sugar derivative having a solubility of at least 100g/100g water at 20°C and 1 atm and the total amount of substrate and solute in the composition is 25 to 60% by weight.

A method of preparing a composition may comprise combining: enzyme that is able to convert a substrate to release hydrogen peroxide; purified substrate for the enzyme; and non-aqueous solvent, wherein the composition is formulated such that the water activity of the composition is 0.7 or less and such that: i) the total amount of sugar or sugar derivative in the composition is 25 to 60% by weight; or ii) the composition comprises solute in the form of a sugar or sugar derivative having a solubility of at least 100g/100g water at 20°C and 1 atm and the total amount of substrate and solute in the composition is 25 to 60% by weight.

In preferred embodiments, the compositions may comprise at least 2% by weight of the non-aqueous solvent, at least 5% by weight of the non-aqueous solvent, at least 10% by weight of the non-aqueous solvent, at least 20% by weight of the non-aqueous solvent at least 30% by weight of the non-aqueous solvent or at least 40% by weight of the non-aqueous solvent. The non-aqueous solvent may be present in an amount of 50% by weight or less, 40% by weight or less, 30% by weight or less, or 20% by weight or less.

For example, there may be 2% to 50% by weight non-aqueous solvent in the composition. There may be 5% to 40% by weight non-aqueous solvent in the composition. There may be 20% to 40% by weight non-aqueous solvent in the composition.

In some embodiments, compositions of the invention may comprise hydrogen peroxide scavenger or free radical scavenger. For example, compositions of the invention may comprise antioxidant. Antioxidant may consist of one antioxidant or may consist of a plurality of antioxidants. Suitable antioxidants include ascorbic acid, tocopherol or ascorbyl palmitate. Ascorbic acid may be preferred. The applicant has found that antioxidants, such as ascorbic acid, may be particularly effective at reducing the amount of hydrogen peroxide in the composition prior to dilution, and resisting reductions in enzyme activity during storage. Antioxidant may be present in an amount of at least 0.01% by weight or at least 0.1% by weight. Antioxidant may be present in an amount of 2% by weight, or less, 1% by weight, or less, or 0.5% by weight, or less. For example, in some embodiments, antioxidant is present in an amount of 0.1 to 0.5% by weight, such as 0.25% by weight.

A composition may comprise: enzyme that is able to convert a substrate to release hydrogen peroxide; purified substrate for the enzyme; solute in the form of a sugar or sugar derivative having a solubility of at least 100g/100g water at 20°C and 1 atm; and antioxidant, wherein the water activity of the composition is 0.7 or less.

A method of preparing a composition may comprise combining: enzyme that is able to convert a substrate to release hydrogen peroxide; purified substrate for the enzyme; solute in the form of a sugar or sugar derivative having a solubility of at least 100g/100g water at 20°C and 1 atm; and antioxidant, wherein the composition is formulated to have a water activity of 0.7 or less.

The concentration of scavenger (e.g. antioxidant) in compositions of the invention may be from 0.01 % by weight to 1% by weight. Preferably, the antioxidant is present in an amount of 0.05% by weight to 0.5% by weight.

More preferably, the antioxidant is present in an amount of at least 0.1%. The antioxidant may be present in an amount less than 0.5%, such as 0.25%, because higher levels of antioxidant may have the potential to suppress hydrogen peroxide production following dilution. For example, it may suppress the initial burst of hydrogen peroxide production following dilution.

Compositions of the invention comprise a polymer. The polymer in the composition may be any medically acceptable polymer, such as any Food and Drug Administration-approved (FDA-approved) polymer.

In some embodiments, the polymer may be a synthetic polymer. In some embodiments, the polymer is a natural polymer.

Optionally, the polymer is water soluble. The polymer may be soluble in an organic, or non-aqueous, solvent. The polymer may be soluble in a mixture of an aqueous and non-aqueous solvent. The polymer may be biodegradable or bioerodable. The polymer may be a co-polymer. In some embodiments, the polymer is selected from polyethylene oxide (or polyethylene glycol), polyvinyl alcohol and polyvinylpyrrolidone.

Other polymers may include poly(lactic-co-glycolic acid), polyglycolic acid, polylactic acid, polycaprolactone or polymeric surfactants. Another suitable polymer may be phosphino-carboxylic acid (PCA).

Further polymers may include polysaccharides such as cellulose (which includes derivatives such as hydroxypropyl methyl cellulose and hydroxypropyl cellulose), alginate, gelatin or cyclodextrins. Suitable polymers may also include chitosan or hyaluronic acid.

Compositions of the invention may comprise up to 50%, 25%, 10% or 5% by weight of the polymer. For example, the composition may comprise from 0.5 to 3% by weight of the polymer. Optionally, the polymer may be from 0.5 to 50% by weight of the composition, such as 5 to 20% by weight of the composition.

Compositions of the invention may be electrospinnable.

There is also provided (not part of the claimed invention) a method of producing a fiber, comprising electrospinning a composition of the invention.

Compositions may be in the form of a fiber. The fiber may be a nanofiber or microfiber.

Also disclosed (not claimed) is a fiber coated with a composition of the invention. The fiber may be a nanofiber or microfiber.

Also disclosed (not claimed) is a wound dressing or nanofibrous mat comprising one or more fibers of the invention.

The electrospinnable composition may comprise one or more electrospinnable components. Any electrospinnable component which facilitates formation of a fiber or dressing according to the invention, may be suitable. Preferably, the one or more electrospinnable component is an electrospinnable polymer. In some embodiments, the polymer may be a synthetic polymer. In some embodiments, the polymer is a natural polymer. In some embodiments, the electrospinnable polymer is selected from polyethylene oxide, polyvinyl alcohol and polyvinylpyrrolidone. Other polymers may include polycaprolactone or phosphino-carboxylic acid (PCA).

The electrospinnable component is preferably biocompatible. Optionally, the electrospinnable component is water soluble. The electrospinnable component may be soluble in an organic, or non-aqueous, solvent. The electrospinnable component may be soluble in a mixture of an aqueous and non-aqueous solvent. Suitable non-aqueous solvents may be, or may comprise, glycerol, dimethyl sulphoxide, ethylene glycol or propylene glycol.

The electrospinnable composition may comprise up to 50%, 25%, 10% or 5% by weight of the electrospinnable component. Optionally, the electrospinnable component may be from 1 to 50% by weight of the composition.

It will be appreciated that the relative amounts of the electrospinnable component and the solvent may be varied to alter the properties of the fibers.

Compositions of the invention may comprise salt.

Compositions of the invention may be in the form of an emulsion. The composition may comprise a first phase (or first liquid, or first component) and a second phase (or second liquid, or second component). However, in one embodiment, the composition is not an emulsion.

The first phase and the second may be immiscible. For example, the first phase may be less polar than the second phase. The first phase may be a non-polar phase such as a lipophilic phase or a hydrophobic phase e.g. an oil. The second phase may be a polar phase, such as an aqueous phase. The second phase may comprise a non-aqueous solvent. Droplets or micelles of the second phase may be dispersed within the first phase.

In some embodiments, first phase is or comprises an oil. In some embodiments, the oil is selected from olive oil, corn oil, vegetable oil, sunflower oil or paraffin oil. In a preferred embodiment, the oil is paraffin oil.

In some embodiments, the second phase is, or comprises glycerol.

In some embodiments, compositions of the invention may comprise an emulsifier. An example of a suitable emulsifier is Polyglycerol polyricinoleate (PGPR).

Examples of compositions such as emulsions and methods of forming such emulsions are described in WO 2017/013448 A1 and WO 2018/091890 A1.

In some compositions of the invention, there is substantially no oil or lipophilic phase. Compositions of the invention preferably do not comprise an emulsion.

Compositions of the invention are preferably sterile. Compositions of the invention may be sterilised by any suitable means. Preferably compositions of the invention have been sterilised by irradiation. The Applicant has found that compositions can retain glucose oxidase activity (and, therefore, the ability to release hydrogen peroxide on dilution) following sterilisation by exposure to gamma irradiation or electron beam irradiation. A suitable level of gamma irradiation is 10-70 kGy, preferably 25-70 kGy, more preferably 35-70 kGy. Alternatively, compositions of the invention may be sterilised by electron beam irradiation. A suitable level or dose of irradiation (e.g. electron beam irradiation) may be 10-100 kGy, preferably 30-80 kGy, more preferably 50-80kGy. The dose may be greater than 35 kGy. The dose may be less than 80 kGy, for example 75 kGy or less.

There is also provided according to the invention a method of sterilising a composition of the invention. Preferably, the method comprises exposing the composition to irradiation, preferably gamma irradiation or electron beam irradiation.

Since ozone has not been authorised by the US FDA for sterilisation of honey-based products for use in wound healing, compositions according to the invention preferably have not been sterilized by ozonation, and do not include ozone, or any components that have been subjected to sterilisation by ozonation.

Compositions of the invention may be in a container or sachet. The container may assist in maintaining the sterility of the composition. Preferably, the container is sealed or airtight. The container may have a removable and/or replaceable cap or seal. The container is preferably opaque.

A composition of the invention may be provided with a dressing material. The dressing material may be coated with the composition. Suitable dressings materials include gauzes, bandages, tissues, films, gels, foams, hydrocolloids, alginates, hydrogels, or polysaccharide pastes, granules, beads or tulle. The composition may be present together with a wound-dressing matrix, such as a collagen or collagen-glycosaminoglycan matrix. The dressing may be a tulle dressing. Compositions in combination with a dressing are preferably sterile, and may be sterilised using irradiation, e.g. gamma irradiation or electron beam irradiation.

Compositions may be in the form of hydrogels. Alternatively, the composition may be in the form of a liquid preparation. Examples of liquid preparations include a syrup, paste, spray, drop, ointment, cream, lotion, oil, liniment, or gels. A typical gel includes an alcoholic gel such as an isopropanol, ethanol, or propanol gel, or a hydrogel.

Compositions of the invention can be used to treat any microbial infection that can be treated by hydrogen peroxide. Examples include infection caused by gram positive bacteria, gram negative bacteria, acid-fast bacteria, viruses, yeasts, parasitic or pathogenic micro-organisms or fungi. For example, infections caused by the following micro-organisms may be treated: *Escherichia coli, Staphylococcus aureus, Pseudomonas aeruginosa, Candida albicans, Propionibacterium acnes, Staphylococcus aureus, Staphylococcus epidermidis, Staphylococcus saprophytics,* Beta haemolytic *Streptococci* Group A or B, *Campylobacter coli, Campylobacter jejuni,* Methicillin Resistant *Staphylococcus Aureus* (MRSA), Methicillin Sensitive *Staphylococcus Aureus* (MSSA), *Botrytis cinerea, Mycobacterium tuberculosis, Cryptosporidium, Plasmodium, Streptococcus pyogenes, Streptococcus zooepidemicus* and *Toxoplasma.*

There is further provided according to the invention a composition of the invention for use in the prevention or treatment of a microbial infection, for example a microbial infection that comprises a biofilm, or a microbe that is capable of forming a biofilm. So, there may be provided a composition of the invention for use in the prevention or treatment of a microbial infection that comprises a biofilm or a microbe that is capable of forming a biofilm. The biofilm may comprise bacteria, fungi and/or viruses.

There is also provided a method of preventing or treating a microbial infection, for example a microbial infection that comprises a biofilm, or a microbe that is capable of forming a biofilm, wherein the method comprises administering an effective amount of a composition of the invention to a site of the infection.

There is also provided use of a composition of the invention to prevent or inhibit microbial growth.

There is also provided according to the invention a composition of the invention for use as a medicament.

Compositions of the invention may be used to treat animals. Compositions of the invention may be used to treat humans.

There is further provided a composition of the invention for the prevention, treatment, or amelioration of a microbial infection.

There is also provided use of a composition of the invention in the manufacture of a medicament for the prevention, treatment, or amelioration of a microbial infection.

There is further provided a method of preventing, treating, or ameliorating a microbial infection, which comprises administering a composition of the invention to a subject in need of such prevention, treatment or amelioration. The subject may be a human or animal subject. Compositions of the invention may be topically administered.

There may be provided compositions of the invention for use in the treatment of a microbial infection that comprises a biofilm.

There is also provided a device for delivering a composition to a patient, the device comprising a composition of the invention.

The device may be a spraying or atomising device, such as a pump-action spray or an aerosol spray. The device may be an inhaler, for example, a metered-dose inhaler, a dry powder inhaler, a nebulizer, for delivery of the composition into the lungs, or a nasal inhaler.

The device may be for external use, such as for applying the composition to a patient's skin.

The device may be for internal application to a patient. For example, the device may be an inhaler or nebuliser for administering the composition to the patient's respiratory tract. Compositions of the invention may thus be used to treat pulmonary infections, such as pulmonary viral infections. The device may be a douche. The device may be a device for injecting the composition into the patient. The device may be a syringe.

Compositions of the invention may be for prophylactic applications.

According to a preferred aspect of the invention, a composition of the invention may be for use in a method of wound care, including the treatment of a wound, or the treatment or management of wound sepsis.

The wound may be an acute wound, chronic wound, surgical wound (for example, a Caesarean wound), chronic burn, or an acute burn. A composition of the invention may be used in the prophylactic prevention of wound sepsis. If a storage-stable composition of the invention is used, it will be appreciated that this may be diluted by liquid present at the wound site, which thereby leads to the release of hydrogen peroxide by the diluted composition.

There is also provided according to the invention a composition of the invention for treatment of a wound. There is also provided a method of treating a wound, which comprises administering a composition of the invention to a subject in need of such treatment. There is also provided a use of a composition of the invention in the manufacture of a medicament for the treatment of a wound.

Compositions of the invention may be used to treat chronic wounds or wounds that are critically colonized. The term "critically colonized" is often used to refer to a wound that has reached a critical point at which bacteria begin to negatively affect the wound and begin to elicit signs of their presence. A critically colonized wound may indicate the presence of a biofilm. A bacterial load of greater than 10⁵ organisms/gram of tissue is often accepted as impeding wound healing (Siddiqui AR, Bernstein JM (2010) Chronic wound infection: Facts and controversies. Clinics in Dermatology 28: 519-26; Edmonds, M., & Foster, A. (2004). The use of antibiotics in the diabetic foot. Am J Surg, 187(5A), 25S-28S. Consequently, compositions of the invention may be used to treat wounds that have a bacterial load of greater than 10⁵ organisms/gram of tissue.

Compositions of the invention may be administered to a patient, such as placed on the wound of a patient, for a period of at least 24 hours or 48 hours or, more preferably, 72 hours.

There is further provided a composition of the invention in the manufacture of a medicament for treatment of a wound.

There is also provided a method of treating inflammation, which comprises administering a composition of the invention to a site of inflammation.

There is also provided a composition of the invention for treatment of inflammation.

There is further provided use of a composition of the invention in the manufacture of a medicament for treatment of inflammation.

There is also provided a method of stimulating tissue growth, which comprises administering a composition of the invention to a site in need of such stimulation.

There is also provided according a composition of the invention for stimulating tissue growth.

There is further provided use of a composition of the invention in the manufacture of a medicament for stimulating tissue growth.

There is also provided a method of debriding a wound, which comprises administering a composition of the invention to a wound in need of debridement.

There is also provided a composition of the invention for debriding a wound.

There is further provided use of a composition of the invention in the manufacture of a medicament for debriding a wound.

There is also provided a method of deodorising a wound, which comprises administering a composition of the invention to a wound in need of deodorising.

There is also provided a composition of the invention for deodorising a wound.

There is further provided use of a composition of the invention in the manufacture of a medicament for deodorising a wound.

A composition of the invention may be provided with instructions for use of the composition. For example, a composition of the invention may be packaged as a kit with the instructions.

Examples are now described with reference to the accompanying drawings in which:
Figure 1 shows a hydrogen peroxide production profile, following dilution, of a formulation comprising 31% glucose, 52% fructose, 16.9% water and 0.1% glucose oxidase;
Figure 2 shows a hydrogen peroxide production profile, following dilution, of a formulation comprising 31% glucose, 52% fructose, 9.9% water, 7% glycerol and 0.1% glucose oxidase;
Figure 3 shows a hydrogen peroxide production profile, following dilution, of a formulation comprising 5% glucose, 78% fructose, 16.9% water and 0.1% glucose oxidase;
Figure 4 shows a hydrogen peroxide production profile, following dilution, of a formulation comprising 5% glucose, 83% fructose, 11.9% water and 0.1% glucose oxidase;
Figure 5 shows the water activities of the formulations of Figures 1 to 4;
Figure 6 shows levels of hydrogen peroxide in formulations comprising 5% glucose, 78% fructose, 9.9% water, 7% glycerol and 0.025% glucose oxidase, both with and without 0.5% ascorbic acid, over a period of 7 days;
Figure 7 shows glucose oxidase activity levels of the formulations of Figure 5, over a period of 7 days; and
Figure 8 shows a hydrogen peroxide production profile, following dilution, of a formulation comprising 5% glucose, 78% fructose, 9.9% water, 7% glycerol and 0.025% glucose oxidase, with both 0.1% ascorbic acid and 0.5% ascorbic acid.

### Example 1

### Methods

Samples were formulated as follows (all amounts are expressed as % by weight).

**Table 1**

| | Glucose | Fructose | Water | Glycerol | Glucose Oxidase |
|---|---|---|---|---|---|
| Sample/formulation 1 | 31 | 52 | 16.9 | 0 | 0.1 |
| Sample/formulation 2 | 31 | 52 | 9.9 | 7 | 0.1 |
| Sample/formulation 3 | 5 | 78 | 16.9 | 0 | 0.1 |
| Sample/formulation 4 | 5 | 83 | 11.9 | 0 | 0.1 |

### Stability study

On the first day of the study the samples were placed at 25°C in the incubator. Each day of the study (day 1, 3, and 6) samples were taken from the incubator, mixed by stirring and then analysed for glucose oxidase activity (TM-601) and hydrogen peroxide levels (TM-781). All dilutions were performed gravimetrically to ensure the highest level of accuracy. In addition on day 1 and 6 each of the samples was activated by the addition of an equal mass of water and incubated at 37°C for up to 72 hours. At specified times the level of hydrogen peroxide was determined by testing an aliquot of the incubated activated solution.

### Glucose oxidase assay (TM-601)

Approximately 1g of samples were weighed into a 15ml tube and diluted with 9 mL of phosphate buffered saline (PBS). All weights were measured using a 5 place balance for calculation of actual dilutions. The samples were vortex mixed to ensure that the entire sample was solubilised and then diluted serially to give a dilution between 5,000 and 100,000 times for analysis. 50 1-JL of sample, standard curve point, blank or quality control was pipetted into a 96 well plate and the reaction started by addition of 50 1-JL of substrate. The change in absorbance at 570 nm was monitored over a half an hour time period and the rate of change of OD used to calculate the glucose oxidase activity by comparison with the standard curve.

### Hydrogen peroxide assay (TM-781)

Approximately 0.3g of samples were weighed into 1.5 ml microfuge tubes and diluted with and equal mass of 99% ethanol. The solutions were physically mixed using a sterile pipette tip prior to being centrifuged to remove precipitated material. The ethanol supernatant was diluted through a series of three dilutions (500 to 10,000 times) with PBS. 50 1-JL of sample, standard curve point or blank was pipetted into a 96 well plate and the reaction started by addition of 50 1-JL of substrate. After five minutes the absorbance at 570 nm was determined and used to produce the standard curve and quantify the level of hydrogen peroxide in each sample.

### Activated hydrogen peroxide level determination

In order to determine the level of hydrogen peroxide when the formulation was activated by contact with water approximately 0.65g of sample was mixed well with an equal mass of water and made homogeneous by vortex mixing. This solution was incubated at 37°C for up to 72 hours. Periodically samples were taken and extracted with 99% ethanol to precipitate the protein and sugar. Then following a dilution with PBS the samples were analyzed for hydrogen peroxide level using the Amplex Red hydrogen peroxide assay (TM-781) as detailed above.

### Results

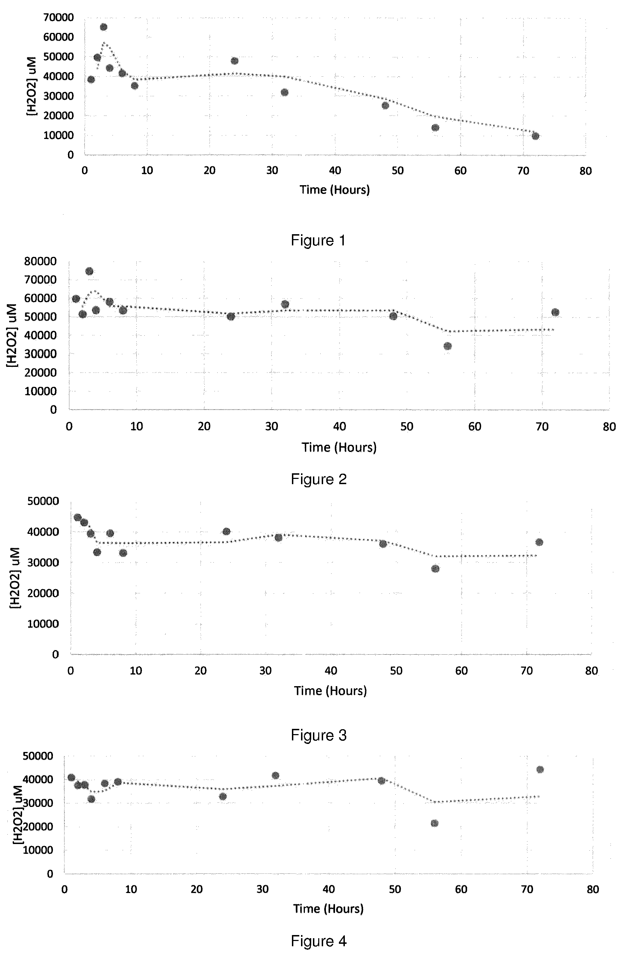

Figures 1, 2, 3 and 4 show graphical representations of the change in hydrogen peroxide concentration with time following activation of samples 1, 2, 3 and 4, respectively, by addition of water.

It can be observed how a significant reduction in the level of glucose can still result in a composition that is able to produce hydrogen peroxide over an extended period of time following dilution.

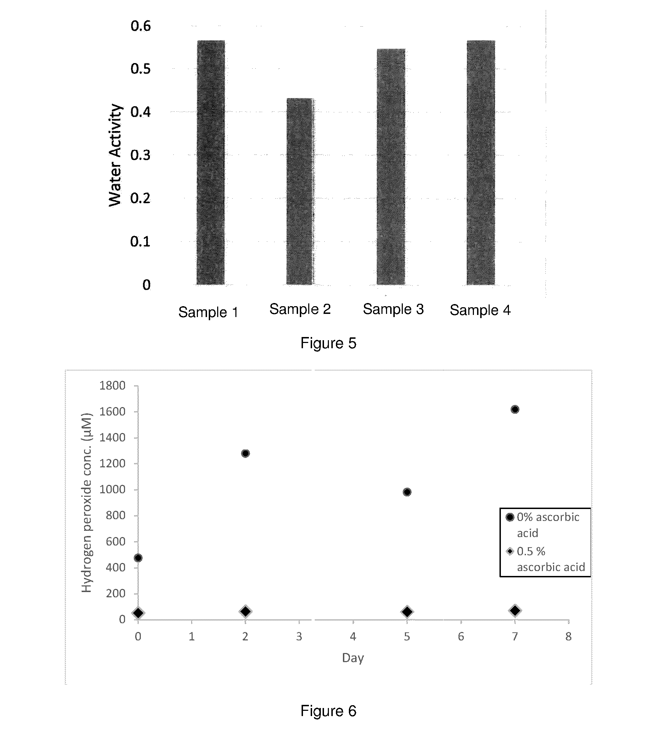

It is noted that the water activity of all samples was less than 0.6 (See figure 5).

It is also noted that Sample 4 was visually biphasic with significant amounts of precipitated sugar.

From the stability study, the following changes in glucose oxidase activity were observed.

**Table 2**

| | % change in glucose oxidase activity (day 1 to day 3) | % change in glucose oxidase activity (day 1 to day 6) |
|---|---|---|
| Sample/Formulation 1 | -9.55 | -22.05 |
| Sample/Formulation 2 | -2.22 | +1.78 |

These data indicates that inclusion of a non-aqueous solvent such as glycerol can reduce loss of glucose oxidase activity.

### Example 2

**Table 3**

| | Glucose | Fructose | Water | Glycerol | Glucose Oxidase |
|---|---|---|---|---|---|
| Sample 1 (Variant 1) | 31 | 52 | 9.9 | 7 | 0.1 |
| Sample 2 (Variant 2) | 5 | 78 | 9.9 | 7 | 0.1 |

| | | | | | |
|---|---|---|---|---|---|
| All amounts % by weight | | | | | |

### Methods

In order to prepare a range of products containing different levels of hydrogen peroxide, the samples were mixed according to the tables below, with the masses being recorded to calculate the exact dilution being performed

### Sample 1:

**Table 4**

| Dilution number | Target [Gox] (%) | Mass of Sample (g) | Description of sample | Mass of variant (g) | Dilution Factor (times) | Actual [GoX] (%) |
|---|---|---|---|---|---|---|
| 1 | 0.05 | 20.59269 | 0.1% GoX variant _ | 20.59868 | 2.0 | 0.0500 |
| 2 | 0.025 | 20.24319 | 0.059% (dilution1) | 20.30243 | 2.0 | 0.0250 |
| 3 | 0.005 | 10.01538 | 0.625% (dilution2) | 40.05496 | 5.0 | 0.0050 |
| 4 | 0.003 | 30.01160 | 0.005% (dilution 3) | 22.10454 | 1.7 | 0.0029 |
| 5 | 0.001 | 10.24168 | 0.003% (ditution4) | 20.12446 | 3.0 | 0.0010 |

### Sample 2:

**Table 5**

| Dilution number | Target [GoX] (%) | Mass of Sample (g) | Description of sample | Mass of variant (g) | Dilution Factor (times) | Actual [GoX] (%) |
|---|---|---|---|---|---|---|
| 6 | 0.05 | 20.90064 | 0.1% GoX variant | 20.12883 | 2.0 | 0.0502 |
| 7 | 0.025 | 20.11057 | 0.05% (dilution 6) | 20.33254 | 2.0 | 0.0250 |
| 8 | 0.005 | 10.39779 | 0.0250% (dilution 7) | 40.43689 | 4.9 | 0.0051 |
| 9 | 0.003 | 30.29630 | 0.005% (dilution 8) | 20.02033 | 1.7 | 0.0031 |
| 10 | | 10.35204 | 0.003% (dilution 9) | 20.06589 | 2.9 | 0.0010 |

Each of these samples was tested for the level of resting hydrogen peroxide using the Amplex Red hydrogen peroxide assay (TM-781, see Example 1), as well as set up for activation by addition of an equal mass of water and incubation at 37ºC for up to 24 hours.

The level of activated hydrogen peroxide was determined as described in Example 1.

**Table 6**

| | Resting [H₂O₂] µM | 1 hour [H₂O₂] µM | Ratio |
|---|---|---|---|
| Sample 1 0.025% GOX | 65.7 | 2425.38 | 369 |
| Sample 1 0.005% GOX | 21.9 | 11170.69 | 510 |
| Sample 1 0.003% GOX | 17.1 | 9506.756 | 557 |
| Sample 1 0.001% GOX | 10.6 | 4582.596 | 432 |
| Sample 2 0.025% GOX | 43.2 | 17097.48 | 395 |
| Sample 2 0.005% GOX | 26.9 | 9800.408 | 364 |
| Sample 2 0.003% GOX | 27.4 | 8444.577 | 308 |
| Sample 2 0.001% GOX | 27.8 | 2842.829 | 102 |

### Example 3

**Table 7**

| | Glucose | Fructose | Water | Glycerol | Glucose Oxidase | Ascorbic Acid |
|---|---|---|---|---|---|---|
| Sample 1 (Variant 1) | 5 | 78 | 9.9 | 7 | 0.1 | 0.5 |
| Sample 2 (Variant 2) | 5 | 78 | 9.9 | 7 | 0.1 | 0.1 |

| | | | | | | |
|---|---|---|---|---|---|---|
| All amounts % by weight | | | | | | |

In order to prepare samples containing different levels of glucose oxidase, the samples were prepared by gravimetric mixing of a 0.1% and 0% glucose oxidase base preparations, with the masses being recorded to calculate exact dilutions used.

Each of the samples plus the supplied 0.1% GoX for each were then tested for the level of resting hydrogen peroxide and glucose oxidase activity using the Amplex Red hydrogen peroxide and glucose oxidase assays (TM-781 and TM-601; see above) respectively.

### Resting and stability hydrogen peroxide and glucose oxidase levels

For glucose oxidase determination, approximately 0.1g of each sample was weighed into 1.5 mL microfuge tubes and diluted with 900µl PBS. For hydrogen peroxide, approximately 0.3g of each sample was weighed into 1.5 ml microcentrifuge tubes and diluted with an equal mass of 99% ethanol. For both, the solutions were physically mixed using a sterile pipette tip prior to being centrifuged to remove precipitated material and then diluted to a suitable level using PBS. 50 µl of sample, standard curve point (hydrogen peroxide or glucose oxidase as required) or blank was pipetted into a 96 well plate and the reaction started by addition of 50µl of the relevant substrate. For both assays, the absorbance at 570 nm was determined, for glucose oxidase, every 2 minutes for a total of 30 minutes and after 5 minutes for hydrogen peroxide. This was then used to produce the standard curve for each assay and quantify both the glucose oxidase activity and hydrogen peroxide level in each sample. The samples were also stored at 25ºC for 7 days and tested at 2, 5 and 7 days thereafter.

### 7 day stability study of 0.25% glucose oxidase formulation containing different levels of ascorbic acid.

**Table 8**

| | Time (days) | | | |
|---|---|---|---|---|
| Ascorbic acid % | | 2 | 5 | 7 |
| 0% ascorbic acid | 476.6 | 1278.8 | 981.3 | 1618.8 |
| 0.5 % ascorbic acid | 50.1 | 61.6 | 59.7 | 69.1 |
| | [H₂O₂] µM | | | |

The results are illustrated in Figure 6. The resting levels of hydrogen peroxide were reduced by the presence of ascorbic acid.

**Table 9**

| | Time (days) | | | |
|---|---|---|---|---|
| Ascorbic acid % | 0 | 2 | 5 | 7 |
| 0% ascorbic acid | 50474.7 | 16252.9 | 1190.1 | 2756.7 |
| 0.5 % ascorbic acid | 77285.3 | 64534.6 | 54056.1 | 34364.4 |
| | GOX activity (mU/ml) | | | |

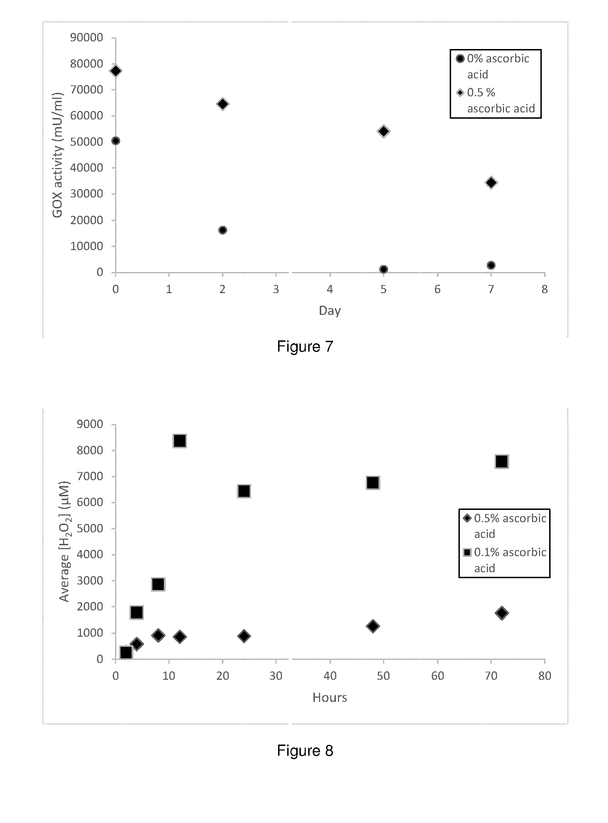

The results are illustrated in Figure 7. In the presence of ascorbic acid, the glucose oxidase has a higher initial activity and glucose oxidase activity is maintained at a higher level over time, over the testing period.

### Simulated wound hydrogen peroxide level determination

A simulated wound was established by weighing 0.5g of the sample into a microfuge tube and centrifuging briefly to collect the gel at the bottom of the tube. On top of this was added 0.5 ml of horse serum and the tube incubated at 30ºC for up to 72 hours. During this time, 10µl samples of the liquid supernatant (after mixing by inversion) were removed into either 140 µl of 99% ethanol for hydrogen peroxide measurement.

The hydrogen peroxide assay (TM-781) vortex mixed the sample briefly, followed by centrifugation at 12,000 rpm for 2 minutes to pellet precipitated material. The ethanol supernatant was further diluted with PBS and tested for hydrogen peroxide levels the Amplex Red assay kit.

### 72 hour hydrogen peroxide profiles

**Table 10**

| | 0.1% ascorbic acid | | 0.5% ascorbic acid | |
|---|---|---|---|---|
| Time (hours) | Average [H202] (µM) | Standard deviation | Average [H202] (µM) | Standard deviation |
| 2 | 247 | 105.5 | 268.1 | 140 |
| 4 | 1777.8 | 801.3 | 573.6 | 16.1 |
| 8 | 2856.7 | 801.3 | 902.1 | 22.1 |
| 12 | 8364.2 | 457 | 851.2 | 127.3 |
| 24 | 6431 | 524.8 | 877.5 | 199.6 |
| 48 | 6754.2 | 326 | 1263.3 | 135.7 |
| 72 | 7568.8 | 46.4 | 1749.8 | 320.7 |
| 0.025% Glucose oxidase | | | | |

The results are illustrated in Figure 8.

## Claims

1. A liquid or gel composition comprising:
enzyme that is able to convert a substrate to release hydrogen peroxide;
substrate for the enzyme, the substrate being less than 10%, by weight, of the composition, preferably 7.5% or less, by weight, of the composition; and
polymer,
wherein the composition does not comprise sufficient free water to allow the enzyme to convert the substrate or has a water activity of 0.7 or less.

2. A composition according to claim 1, wherein the substrate is at least 1%, by weight, of the composition, preferably at least 2%, by weight, of the composition.

3. A composition according to claim 1 or claim 2, comprising solute in the form of a sugar, with a solubility of at least 300g/100g water at 20°C and 1 atm.

4. A composition according to any of claim 3, wherein the solute is, or comprises, fructose.

5. A composition according to any preceding claim, which does not comprise honey.

6. A composition according to any preceding claim, comprising water in an amount of 5% or less, by weight.

7. A composition according to any preceding claim, which comprises substantially no hydrogen peroxide, wherein hydrogen peroxide is present at a concentration of 6 ppm or less, or wherein the hydrogen peroxide is present at a concentration of 3 ppm or less.

8. A composition according to any preceding clam, wherein the polymer is polyethylene glycol.

9. A composition according to any preceding claim, wherein the enzyme is glucose oxidase and the substrate for the enzyme is D-glucose.

10. A composition according to any preceding claim which is sterile.

11. A composition according to any preceding claim, wherein the enzyme is 0.001% to 0.5%, by weight, of the composition or 0.0025% to 0.2%, by weight, of the composition.

12. A composition according to any of claims 1 to 11, for use as a medicament, optionally for use in prevention, treatment or amelioration of a microbial infection, or for use in treatment of a wound.

13. A method of preparing a composition, the method comprising, combining: an enzyme that is able to convert a substrate to release hydrogen peroxide; a substrate for the enzyme; and polymer, wherein the composition is formulated to have a water activity of 0.7 or less, or such that it does not include sufficient free water to allow the enzyme to convert the substrate, wherein the composition is formulated such that it is a liquid or gel, and wherein substrate is added such that it is less than 10% by weight of the composition.

14. A method according to claim 13, wherein the enzyme and substrate are purified, preferably with a mass purity of at least 95%, or at least 98%.

15. A method according to claim 13 or claim 14, comprising sterilising such that the composition is sterile, optionally comprising sterilising by irradiation, preferably using gamma irradiation or electron beam irradiation.

## Patentansprüche

1. Flüssige oder gelartige Zusammensetzung, die Folgendes umfasst:
ein Enzym, das ein Substrat umwandeln kann, um Wasserstoffperoxid freizusetzen;
ein Substrat für das Enzym, wobei das Substrat weniger als 10 Gew.-% der Zusammensetzung, vorzugsweise 7,5 Gew.-% oder weniger der Zusammensetzung ausmacht; und
ein Polymer,
wobei die Zusammensetzung nicht genügend freies Wasser enthält, damit das Enzym das Substrat umwandeln kann, oder eine Wasseraktivität von 0,7 oder weniger aufweist.

2. Zusammensetzung nach Anspruch 1, wobei das Substrat mindestens 1 Gew.-% der Zusammensetzung, vorzugsweise mindestens 2 Gew.-% der Zusammensetzung ausmacht.

3. Zusammensetzung nach Anspruch 1 oder Anspruch 2, das einen gelösten Stoff in Form eines Zuckers mit einer Löslichkeit von mindestens 300 g/100 g Wasser bei 20 °C und 1 atm umfasst.

4. Zusammensetzung nach Anspruch 3, wobei der gelöste Stoff Fructose ist oder umfasst.

5. Zusammensetzung nach einem vorherigen Anspruch, die keinen Honig umfasst.

6. Zusammensetzung nach einem vorherigen Anspruch, die Wasser in einer Menge von 5 Gew.-% oder weniger umfasst.

7. Zusammensetzung nach einem vorherigen Anspruch, die im Wesentlichen kein Wasserstoffperoxid umfasst, wobei Wasserstoffperoxid in einer Konzentration von 6 ppm oder weniger vorliegt oder wobei das Wasserstoffperoxid in einer Konzentration von 3 ppm oder weniger vorliegt.

8. Zusammensetzung nach einem vorherigen Anspruch, wobei das Polymer Polyethylenglykol ist.

9. Zusammensetzung nach einem vorherigen Anspruch, wobei das Enzym Glucoseoxidase ist und das Substrat für das Enzym D-Glucose ist.

10. Zusammensetzung nach einem vorherigen Anspruch, die steril ist.

11. Zusammensetzung nach einem vorherigen Anspruch, wobei das Enzym 0,001 bis 0,5 Gew.-% der Zusammensetzung oder 0,0025 bis 0,2 Gew.-% der Zusammensetzung ausmacht.

12. Zusammensetzung nach einem der Ansprüche 1 bis 11 zur Verwendung als Arzneimittel, optional zur Verwendung bei der Vorbeugung, Behandlung oder Linderung einer mikrobiellen Infektion oder zur Verwendung bei der Behandlung einer Wunde.

13. Verfahren zur Herstellung einer Zusammensetzung, wobei das Verfahren das Kombinieren beinhaltet von: einem Enzym, das ein Substrat umwandeln kann, um Wasserstoffperoxid freizusetzen; einem Substrat für das Enzym; und einem Polymer, wobei die Zusammensetzung so formuliert ist, dass sie eine Wasseraktivität von 0,7 oder weniger aufweist, oder so, dass sie nicht genügend freies Wasser enthält, damit das Enzym das Substrat umwandeln kann, wobei die Zusammensetzung so formuliert ist, dass sie eine Flüssigkeit oder ein Gel ist, und wobei das Substrat so zugegeben wird, dass es weniger als 10 Gew.-% der Zusammensetzung ausmacht.

14. Verfahren nach Anspruch 13, wobei das Enzym und das Substrat gereinigt werden, vorzugsweise mit einer Massenreinheit von mindestens 95 % oder mindestens 98 %.

15. Verfahren nach Anspruch 13 oder Anspruch 14, das Sterilisieren beinhaltet, so dass die Zusammensetzung steril ist, und optional das Sterilisieren durch Bestrahlung, vorzugsweise mit Gammastrahlung oder Elektronenstrahlbestrahlung, beinhaltet.

## Revendications

1. Composition liquide ou de gel comprenant :
une enzyme qui est capable de convertir un substrat pour dégager du peroxyde d'hydrogène ;
un substrat pour l'enzyme, le substrat faisant moins de 10 % en poids de la composition, de préférence 7,5 % en poids ou moins de la composition ; et un polymère
où la composition ne comprend pas suffisamment d'eau libre pour permettre à l'enzyme de convertir le substrat ou a une activité d'eau de 0,7 ou moins.

2. Composition selon la revendication 1, dans laquelle le substrat fait au moins 1 % en poids de la composition, de préférence au moins 2 % en poids de la composition.

3. Composition selon la revendication 1 ou la revendication 2, comprenant un soluté sous la forme d'un sucre ayant une solubilité d'au moins 300 g/100 g d'eau à 20 °C et 1 atm.

4. Composé selon la revendication 3, dans laquelle le soluté est ou comprend du fructose.

5. Composition selon l'une quelconque des revendications précédentes, qui ne comprend pas de miel.

6. Composition selon l'une quelconque des revendications précédentes, comprenant de l'eau en une quantité de 5 % en poids ou moins.

7. Composition selon l'une quelconque des revendications précédentes, qui ne comprend sensiblement pas de peroxyde d'hydrogène, dans laquelle le peroxyde d'hydrogène est présent à une concentration de 6 ppm ou moins, ou dans laquelle le peroxyde d'hydrogène est présent à une concentration de 3 ppm ou moins.

8. Composition selon l'une quelconque des revendications précédentes, dans laquelle le polymère est du polyéthylène glycol.

9. Composition selon l'une quelconque des revendications précédentes, dans laquelle l'enzyme est de la glucose oxydase et le substrat pour l'enzyme est du D-glucose.

10. Composition selon l'une quelconque des revendications précédentes qui est stérile.

11. Composition selon l'une quelconque des revendications précédentes, dans laquelle l'enzyme fait de 0,001 % à 0,5 % en poids de la composition ou de 0,0025 % à 0,2 % en poids de la composition.

12. Composition selon l'une quelconque des revendications 1 à 11, pour utilisation comme un médicament, optionnellement pour utilisation dans la prévention, le traitement ou l'amélioration d'une infection microbienne, ou pour utilisation dans le traitement d'une plaie.

13. Procédé de préparation d'une composition, le procédé comprenant combiner : une enzyme qui est capable de convertir un substrat pour dégager du peroxyde d'hydrogène ; un substrat pour l'enzyme ; et un polymère, dans lequel la composition est formulée pour avoir une activité d'eau de 0,7 ou moins, ou de sorte qu'elle ne contient pas suffisamment d'eau libre pour permettre à l'enzyme de convertir le substrat, dans lequel la composition est formulée de sorte que c'est un liquide ou un gel et dans lequel le substrat est ajouté de manière à faire moins de 10 % en poids de la composition.

14. Procédé selon la revendication 13, dans lequel l'enzyme et le substrat sont purifiés, de préférence avec une pureté de masse d'au moins 95 % ou d'au moins 98 %.

15. Procédé selon la revendication 13 ou la revendication 14, comprenant stériliser de manière à ce que la composition soit stérile, comprenant optionnellement stériliser par irradiation, de préférence par irradiation gamma ou irradiation par faisceau d'électrons.
